(19) Europäisches Patentamt
European Patent Office
Office européen des brevets

(11) Publication number : **0 390 515 B1**

(12) **EUROPEAN PATENT SPECIFICATION**

(45) Date of publication of patent specification : **02.02.94 Bulletin 94/05**

(51) Int. Cl.$^5$ : **C07D 473/34,** A01N 43/54

(21) Application number : **90303277.9**

(22) Date of filing : **28.03.90**

# (54) Novel adenine derivatives and their use as agents for increasing crop yields.

(30) Priority : **28.03.89 JP 73762/89**
**22.09.89 JP 244919/89**

(43) Date of publication of application : **03.10.90 Bulletin 90/40**

(45) Publication of the grant of the patent : **02.02.94 Bulletin 94/05**

(84) Designated Contracting States : **DE ES FR GB IT**

(56) References cited :
**GB-A- 794 540**
**US-A- 2 966 488**
**US-A- 4 751 292**
**PATENT ABSTRACTS OF JAPAN, unexamined applications, C section, vol. 2, no. 31, February 27, 1978 THE PATENT OFFICE JAPANESE GOVERNMENT page 4294 C 77**

(73) Proprietor : **MITSUBISHI GAS CHEMICAL COMPANY, INC.**
**5-2, Marunouchi 2-chome Chiyoda-Ku**
**Tokyo, 100 (JP)**

(72) Inventor : **Oritani, Takashi**
**3-12 Fuchumachi-Nemunoki, Nei-gun**
**Toyama 939-27 (JP)**
Inventor : **Maruyama, Taketo**
**5-10 Taihei 1-chome**
**Niigata-shi, Niigata 950 (JP)**
Inventor : **Oda, Mitsunori**
**3412-2 Matsuhamacho**
**Niigata-shi, Niigata 950-31 (JP)**
Inventor : **Suzuki, Takashi**
**9-34 Kouei 1-chome**
**Niigata-shi, Niigata 950 (JP)**
Inventor : **Tanaka, Akinori**
**3-1, Takaramachi**
**Niigata-shi, Niigata 950 (JP)**
Inventor : **Yoshinaka, Shigeo**
**2-6, Matsuzono 1-chome**
**Niigata-shi, Niigata 950 (JP)**
Inventor : **Kajita, Toshio**
**2-40, Moto-Ookubo, 4-chome**
**Narashino-shi, Chiba 275 (JP)**
Inventor : **Furushima, Masakazu**
**134-165, Kita**
**Nagareyama-shi, Chiba 270-01 (JP)**

(74) Representative : **Kearney, Kevin David Nicholas et al**
**KILBURN & STRODE 30 John Street**
**London, WC1N 2DD (GB)**

EP 0 390 515 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel adenine derivatives represented by the following formula and salts thereof and to their use as agents for increasing yield of crops which contain one of these adenine derivatives and/or salts thereof as an active ingredient

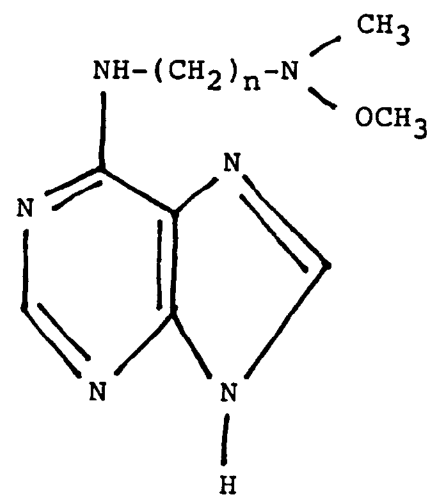

(wherein n is an integer of from 2-4).

Hitherto, many substances have been found for adjustment of growth of plants such as crops, fruits and vegetables. Among them, those which belong to a group of substances generically called cytokinin-active substances are known to have many physiological actions, and representative compounds are zeatin, kinetin and 6-benzylaminopurine (hereinafter referred to as benzyladenine).

Such cytokinin-active substances have been known to have various physiological actions for plants such as the control of growth by cell division in callus and intact plants, the promotion of buds and flower formation, the stimulation of seed germination, the overcoming of dormant phase of seeds, storage organs and buds, the promotion of sideshoot formation, and the retardation of the ageing processes in plants.

Several substances which show cytokinin activity occur naturally, but such naturally occurring substances are limited in their amount and besides are troublesome to obtain. In addition to those natural cytokinins, various synthetic adenine derivatives have been known as substances having cytokinin activity, however their practical use is quite limited. One of the reasons for limitation in their practical use is that not only natural cytokinins but also hitherto known synthetic cytokinins have poor water-solubility and absorption into plants and insufficient translocation to the organs.

Yet to date various studies have been made in attempts to increase the yield of cereal crops such as waterfield rice plants and beans, but few of them have been practically applied in spite of many efforts.

It is an object of the present invention to provide active ingredients which have higher water-solubility than conventional cytokinin-active substances, are high in physiological activity for practical use and easy to manufacture.

It has been known that cytokinins such as kinetin, zeatin, benzyladenine and thidiazuron have activities such as the control of growth by cell division in callus and intact plants, differentiation of plant tissues, stimulation of seed germination and senescence retardation. Recently, an attempt has been made to increase the yield of cereal crops such as waterfield rice plants with the above-mentioned cytokinins. For example, it is reported in "Japanese Journal of Crop Science", Vol. 51, Extra issue 1, 1982, page 147-148 that zeatin and abscisic acid have respectively activity to increase the ripening rate and the thousand-kernel weight of husked rice according to the experiments of direct injection of these plant hormones into the ears of rice plants. More recently Japanese Patent Kokai No. 63-181933 discloses that the yield of gramineous crops can be increased by treating the ears thereof with benzyladenine, thidiazuron, kinetin or zeatin.

Natural cytokins such as zeatin and ribosylzeatin are difficult to manufacture. Benzyladenine, kinetin and thidiazuron can be synthesized with relative ease, but have a defect in water-solubility and have poor absorption into plants and translocation to other organs. Thus, these are not necessarily sufficient for practical use.

As a result of extensive and intensive research conducted by the inventors under the above circumstance, it has been found that adenine derivatives having a side chain represented by the following formula (A) on the $N^6$ nitrogen atom of adenine and salts thereof are effective for attaining the above object. The present invention

is based on such new finding.

Formula (A):

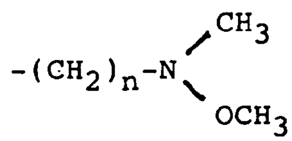

(A)

It has also been found that among these adenine derivatives and salts thereof, $N^6$-[2-(N-methoxy-N-methylamino)ethyl]adenine and salts thereof exhibit especially strong cytokinin activity.

The inventors have further conducted studies on the practical actions of $N^6$-[2-(n-methoxy-N-methylamino)ethyl]adenine and salts thereof on plants and as a result have found that this compound not only has strong cytokinin activity but also increases the yield of agricultural crops when applied to the leaves and stems of leguminous plants and cereal plants such as gramineous plants.

The present invention is also based on these new findings.

The present invention relates to novel adenine derivatives and salts thereof. The adenine derivatives and salts thereof according to the present invention are water-soluble and show excellent cytokinin activity, and therefore are useful for regulation of physiological actions of plants such as the control of growth by cell division in callus and intact plants, the promotion of buds and flower formation, the stimulation of seed germination, the overcoming of dormant phase of seed, storage organs and buds, the promotion of sideshoot formation, and the retardation of the ageing process in plants. Furthermore, the present invention relates to agents, which contain the above novel adenine derivatives and salts thereof as an active ingredient, for increasing yield of crops and to a method for increasing yield of crops.

The invention may be put into practice in various ways and certain specific embodiments will be described by way of example with reference to the accompanying drawings, in which:

Figure 1, Figure 2 and Figure 3 show IR absorption spectra of $N^6$-[2-(N-methoxy-N-methylamino)ethyl]adenine, $N^6$-[3-(N-methoxy-N-methylamino)propyl]adenine, and $N^6$-[4-(N-methoxy-N-methylamino)butyl]adenine of the present invention, respectively;

Figure 4 shows an IR absorption spectrum of $N^6$-[2-(N-methoxy-N-methylamino)ethyl]adenine hydrochloride of the present invention; and

Figure 5 is a graph which shows the $CO_2$ absorption rate of $N^6$-[2-(N-methoxy-N-methylamino)ethyl]adenine of the present invention.

The present invention relates to adenine derivatives represented by the following formula (I) and salts thereof represented by the following formula (II):

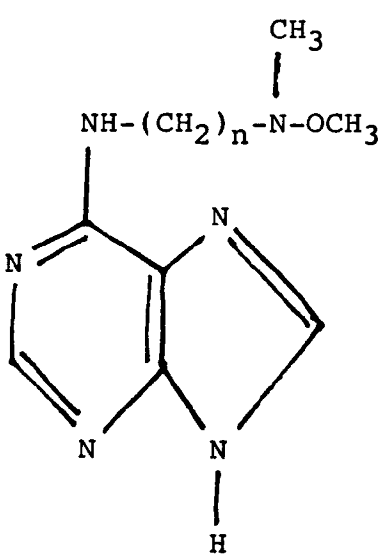

(I)

(wherein n is an integer of 2 to 4).

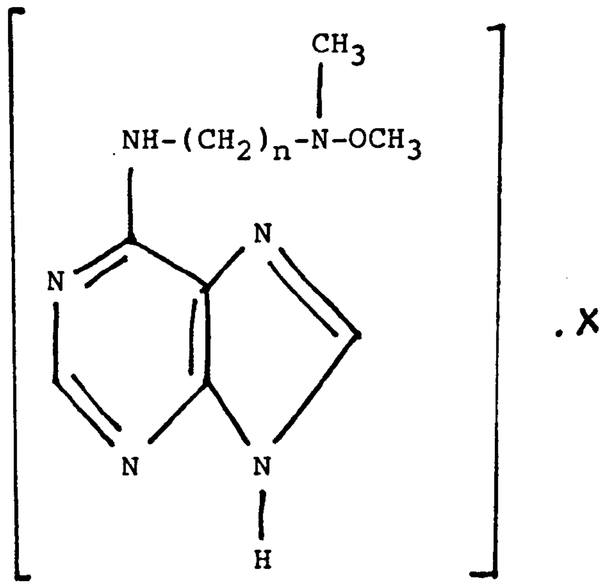

(II)

(wherein n is an integer of from 2 to 4 and X is an equivalent of hydrochloric acid, sulfuric acid, nitric acid, phosphoric acid, formic acid or acetic acid).

Furthermore, the present invention relates to agents for increasing yield of crops which contain $N^6$-[2-(N-methoxy-N-methylamino)ethyl]adenine or salts thereof as an active ingredient.

$N^6$-[2-(N-methoxy-N-methylamino)ethyl]adenine (sometimes referred to as "MAED" hereinafter) has the following structural formula:

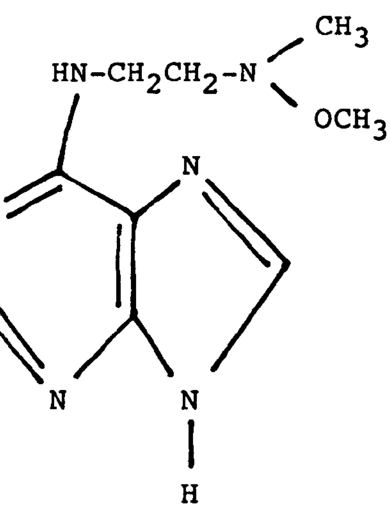

MAED easily forms salts with acids such as hydrochloric acid, sulfuric acid, nitric acid, phosphoric acid, formic acid and acetic acid. MAED and salts thereof are sometimes generically called "MAED's" hereinafter.).

The adenine derivatives presented by the formula (I) can be prepared, for example, by the following procedure.

That is, the adenine derivatives can be synthesized by allowing 6-chloropurine to react with the diamine derivatives represented by the following formula (B) in an organic solvent such as alcohol in the presence of trialkylamine such as ethyldiisopropylamine by heating.

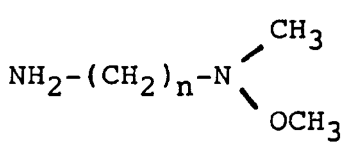

(B)

(wherein n is an integer of from 2 to 4).

In order to obtain the adenine derivatives in high purity, it is important to prepare the diamine derivatives in high purity. These diamine derivatives are preferably prepared as follows. N,O-Dimethylhydroxylamine was allowed to react with N-(haloalkyl)phthalimide to give the phthalimide derivative of the diamine and this phthalimide was deprotected to give the diamine derivative. It is also possible to allow the isolated diamine derivative or the crude diamine derivative to react with 6-chloropurine.

Typical examples of the adenine derivatives represented by the formula (I) are $N^6$-[2-(N-methoxy-N-methylamino)ethyl]adenine represented by the formula (1), $N^6$-[3-(N-methoxy-N-methylamino)propyl]adenine represented by the formula (2) and $N^6$-[4-(N-methoxy-N-methylamino)butyl]adenine represented by the formula (3).

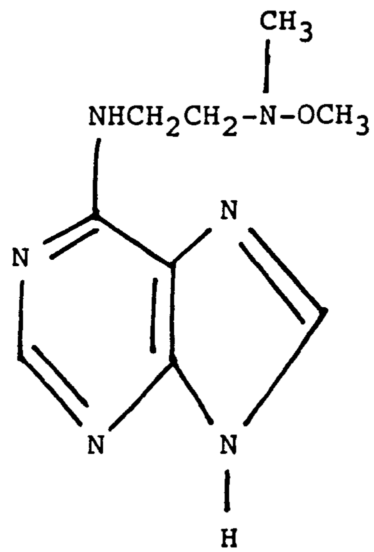

(1)

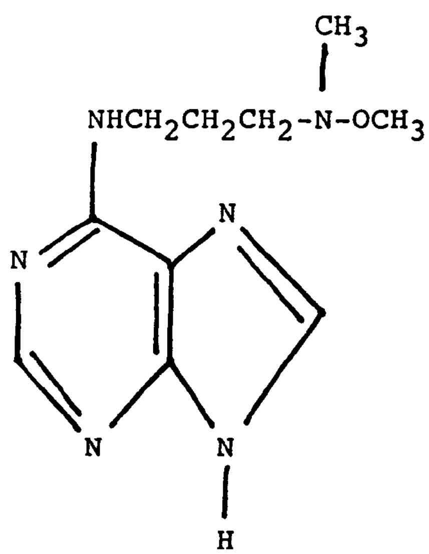

(2)

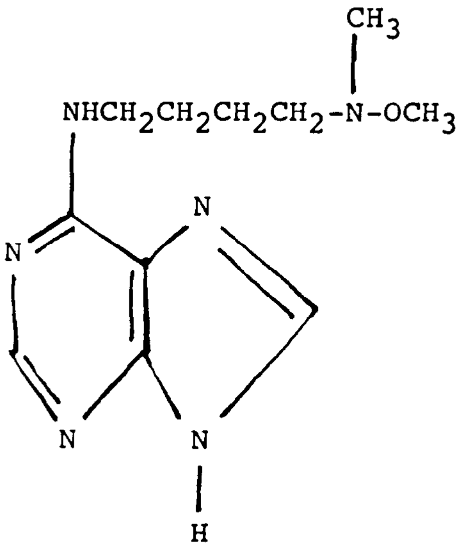

(3)

The adenine derivatives of the present invention can be easily used in the form of salts with mineral acids, such as hydrochloric acid, sulfuric acid, nitric acid or phosphoric acid or salts with organic acids such as formic acid or acetic acid by a conventional procedure.

The salts of the novel adenine derivatives of the present invention are represented by the following formula (II):

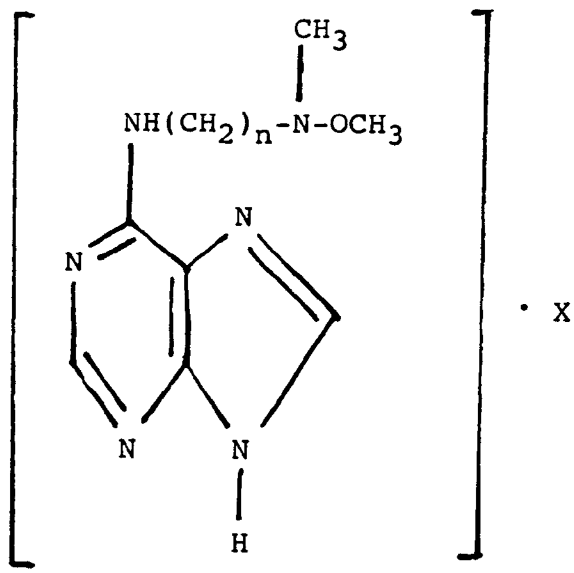

(II)

(wherein n is an integer of from 2-4 and X represents an equivalent of hydrochloric acid, sulfuric acid, nitric acid, phosphoric acid, formic acid or acetic acid).

An example of one of the salts is the hydrochloride of $N^6$-[2-(N-methoxy-N-methylamino)ethyl]adenine represented by the following formula (4):

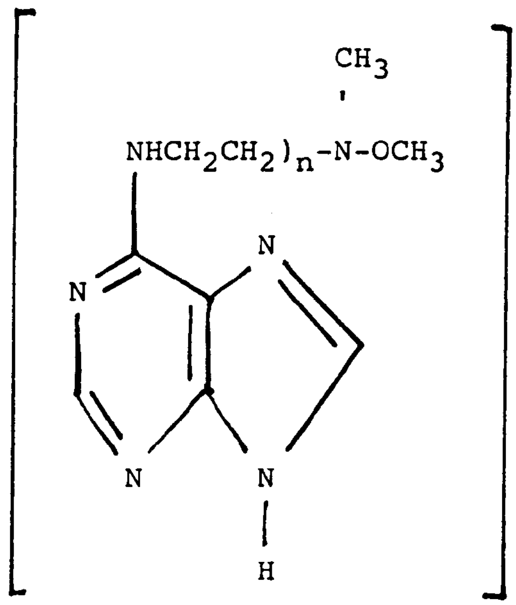

• HCl (4)

The adenine derivatives and salts thereof represented by the above formulas (I) and (II), respectively, have cytokinin activity and hence can be effectively used as plant regulators or for media for plant culture.

MEAD's per se alone can be used as agents for increasing yield of crops, but ordinarily, are used in combination with liquid or solid diluents (a solid diluent is sometimes referred to as a "carrier" hereinafter) and, if necessary, with surface active agents such as emulsifiers, dispersants and spreaders, and stickers which are generally used in the field of formulation of agrochemicals.

Examples of formulations are emulsifiable concentrates, wettable powders, dusts and liquids.

As diluents for preparation of a liquid formulation, mention may be made of polar solvents such as alcohols, e.g. methanol and ethanol, water, dimethylformamide and dimethylsulfoxide.

As carriers for the preparation of wettable powders, granules or dusts, there may be used talc, clay, bentonite, kaolin, montmorilonite, diatomaceous earth, phenol resin and white carbon (calcium silicate).

Typical examples of surface-active agents are anionic surface-active agents such as sodium alkylbenzenesulfonates and sodium laurylsulfate, cationic surface-active agents such as stearyltrimethylammonium chloride and nonionic surface-active agents such as polyoxyethylenealkylphenyl ether.

As dispersants, there may be used, in addition to the above surface-active agents, for example, sodium ligninsulfonate, methylcellulose, and sulfite waste liquor.

As stickers, there may be used, for example, casein lime, glue, carboxymethylcellulose, sodium alginate or polyvinyl alcohol.

Increase of crop yield can be attained by applying the agents.

The parts of the plants to which the agents are best applied, the dosage and the time of application of the agents of the present invention may vary depending on the kind of plants and cannot be simply specified, but an explanation will be given for leguminous plants and gramineous plants.

That is, the agents are applied to the leaves and stems in the case of cereal crops such as leguminous crops or cereal crops such as gramineous crops and the dosage is usually about 0.003-3g/10a, preferably 0.01-1 g/10a in terms of MAED (1a = 100 $m^2$).

The time of application for leguminous crops is usually during the period from the flower bud appearing stage to the grain ripening stage and preferably is during the period of from the beginning of flowering to the initial grain ripening stage. The time of application for gramineous crops is usually during the period of from the panicle formation stage to the ripening stage and preferably is during the period of from the bottom (ear sprouting) stage to the initial grain ripening stage.

Typical examples of crops to which the agents of the present invention may be applied are leguminous crops such as soybean, red bean, kidney bean (Phaseolus vulgaris), winged bean (Psophocarpus) and peanut and gramineous crops such as rice, wheat, barley, rye, pearl barley, oat and maize.

The adenine derivatives and salts thereof of the present invention show excellent cytokinin activity and besides are high in water-solubility and can be employed as a cytokinin substance for a wide variety of agricultural uses and for media of plant tissue culture.

In addition, the agents for increasing yield of crops according to the present invention exhibit remarkable

activity to increase the yield of crops, especially cereal crops.

The invention may be put into practice in various ways and a number of specific embodiments will be described to illustrate the invention with reference to the following nonlimited examples.

## EXAMPLE 1

Preparation of $N^6$-[2-(N-methoxy-N-methylamino)ethyl]adenine of the formula (2):

First, a side chain amine was prepared and then was allowed to react with 6-chloropurine in the following manner.

(1) Preparation of N-[2-(N-methoxy-N-methylamino)-ethyl]phthalimide:

To a suspension of 50 ml of isopropanol containing N,O-dimethylhydroxylamine hydrochloride (3.90 g, 40.0 mmol) was added dropwise 5.56 ml of triethylamine. The resulting mixture became homogeneous after 5 minutes. Thereto was added 2.45 g (10.0 mmol) of N-(2-bromoethyl)phthalimide and the mixture was refluxed for 29 hours. This was cooled to room temperature, then poured into 200 ml of saturated aqueous solution of sodium bicarbonate and extracted with chloroform. The organic layer was separated and was washed with water and dried over magnesium sulfate. Chloroform was distilled off and the residue was purified by column chromatography on silica gel. Development with 30% hexane/chloroform resulted in elution of 1.37 g of N-(2-chloroethyl)phthalimide and further development with chloroform resulted in 0.964 g (yield: 41%) of white crystals of N-[2-(N-methoxy-N-methylamino)ethyl]phthalimide.

Melting point: 130-132°C

$^1H$-NMR (60MHz, $CDCl_3$);

= 2.40 (s, 3H), 2.93(t, J= 6Hz, 2H),
3.53 (s, 3H), 3.93(t, J= 6Hz, 2H),
3.77 (M, 4H) ppm

(2) Preparation of $N^6$-[2-(N-methoxy-N-methylamino)ethyl]adenine by deprotection of the imide, followed by condensation with 6-chloropurine:

0.89 g (3.71 mmol) of the imide obtained in the above Example 1 preparation (1) was dissolved in ethanol and thereto was added 0.204 g (4.08 mmol) of hydrazine hydrate, followed by refluxing for 3 hours and cooling in an ice bath. The resulting solid was filtered off and the filtrate was concentrated to 2 ml by an evaporator in a water bath at 5°C. This concentrated solution was mixed with 5 ml of n-butanol and to the mixture were added 0.200 g (4.2 mmol) of 6-chloropurine and 0.4 ml (4.2 mmol) of ethyldiisopropylamine and the mixture was gently refluxed for 5 hours. This was cooled to room temperature and then the solvent was distilled off under reduced pressure. The residue was purified by column chromatography on silica gel (eluent: chloroform/ethanol = 95/5) to give 0.210 g (yield: 25%) of white crystals of $N^6$-[2-(N-methoxy-N-methylamino)ethyl]adenine.

Melting point: 168-170°C

Elemental Analysis :

| | |
|---|---|
| Molecular formula: | $C_9H_{14}N_6O$ |
| Calculated values: | C; 48.63, H, 6.34, N; 37.81 |
| Found | C; 48.51, H, 6.11, N; 38.10 |

$^1H$-NMR (60 MHz, $CDCl_3$);

= 2.67 (s, 3H), 2.78 (t, J=6Hz, 2H),
3.56 (s, 3H), 3.74 (t, J=6Hz, 2H),
8.05 (s, 1H), 8.12 (s, 1H) ppm

Its IR absorption spectrum (KBr method) is shown in Figure 1.

$\nu_{max}$ = $3350^s$, $1630^s$, $1090^s cm^{-1}$

UV absorption spectrum:

$\lambda$ max = ($H_2O$), 207, 267nm
(0.1 N-HCl ), 275nm
(0.1 N-NaOH), 274nm, $282^{sh}$nm

## EXAMPLE 2

Preparation of $N^6$-[3-(N-methoxy-N-methylamino)propyl]adenine of the formula (3):

First, a side chain amine was prepared and then was allowed to react with 6-chloropurine in the following manner.

(1) Preparation of N-[3-(N-methoxy-N-methylamino)-propyl]phthalimide:

To 50 ml of an isopropanol suspension of N,O-dimethylhydroxylamine hydrochloride (3.97 g, 40.0

mmol) was added dropwise 5.56 ml of triethylamine. After 5 minutes, thereto was added 2.68 g (10.0 mmol) of N-(3-bromopropyl)phthalimide and the mixture was refluxed for 33 hours. This was cooled to room temperature, then poured into 200 ml of a saturated aqueous solution of sodium bicarbonate and extracted with chloroform. The organic layer was separated and was washed with water and dried over magnesium sulfate. Chloroform was distilled off and the residue purified by column chromatography on silica gel. Development with 30% hexane/chloroform resulted in elution of 1.03 g of N-(3-chloropropyl)phthalimide and further development with chloroform resulted in 1.21 g (yield: 53%) of white crystals of N-[3-(N-methoxy-N-methylamino)propyl]-phthalimide.

Melting point: 55-57°C

$^1$H-NMR (60MHz, $CDCl_3$);

= 1.93 (quin, J=6Hz, 2H),
2.55 (s, 3H), 2.68 (t, J=6Hz, 2H),
3.52 (s, 3H), 3.08 (t, J=6Hz, 2H),
7.75 (m, 4H) ppm

(2) Preparation of $N^6$-[3-(N-methoxy-N-methylamino)propyl]adenine by deprotection of the imide, followed by condensation with 6-chloropurine:

1.21 g (4.88 mmol) of the imide obtained in the above Example 2 preparation (1) was dissolved in 15 ml of ethanol and thereto was added 0.293 g (5.86 mmol) of hydrazine hydrate, followed by refluxing for 6 hours and cooling in an ice bath. The resulting solid was filtered off and the filtrate was concentrated to 2 ml by an evaporator with a water bath at 5°C. This concentrated solution was mixed with 6 ml of n-butanol and to the mixture were added 0.528 g (3.42 mmol) of 6-chloropurine and 0.68 ml of ethyldiisopropylamine and the mixture was gently refluxed for 5 hours. This was cooled to room temperature and then the solvent was distilled off under reduced pressure. The residue was purified by column chromatography on silica gel (eluent: chloroform/ethanol = 9/1) to give 0.421 g (yield: 36%) of white crystals of $N^6$-[3-(N-methoxy-N-methylamino)propyl]adenine.

Melting point: 183-185°C

Elemental Analysis:

| | |
|---|---|
| Molecular formula: | $C_{10}H_{16}N_6O$ |
| Calculated values: | C; 50.83, H; 6.83, N; 35.57 |
| Found : | C; 51.02, H, 6.79, N, 35.29 |

$^1$H-NMR (60 $MH_z$, $CDCl_3$);

= 1.96 (quin, J=7Hz, 2H),
2.59 (s, 3H), 2.79 (t, J=7Hz, 2H),
3.53 (s, 3H), 3.71 (t, J=7Hz, 2H),
7.99 (s, 1H), 8.22 (s, 1H) ppm

The IR absorption spectrum (KBr method) is shown in Figure 2.

$\nu_{max}$ = $2930^m$, $1590^s$, $1025^w cm^{-1}$

UV absorption spectrum:

$\lambda_{max}$ = ($H_2O$), 210, 267nm
(0.1 N-HC ), 273nm
(0.1 N-NaOH), 274nm, $284^{sh}$nm

## EXAMPLE 3

Preparation of $N^6$-[4-(N-methoxy-N-methylamino)butyl]adenine of the formula (4):

First, a side chain amine was prepared and then was allowed to react with 6-chloropurine in the following manner.

(1) Preparation of N-[4-(N-methoxy-N-methylamino)butyl]phthalimide:

To a suspension of 50 ml of isopropanol containing N,O-dimethylhydroxylamine hydrochloride (3.97 g, 40.0 mmol) was added dropwise 5.56 ml of triethylamine. The resulting mixture became homogeneous after 5 minutes. Thereto was added 2.00 g (7.09 mmol) of N-(4-bromobutyl)phthalimide and the mixture was refluxed for 58 hours. This was cooled to room temperature, then poured into 200 ml of a saturated aqueous solution of sodium bicarbonate and extracted with chloroform. The organic layer was separated and was washed with water and dried over magnesium sulfate. Chloroform was distilled off and the residue was purified by column chromatography on silica gel. Development with 30% hexane/chloroform resulted in elution of 0.719 g of N-(4-chlorobutyl)phthalimide and further development with chloroform resulted in 0.964 g (yield: 41%) of N-[4-(N-methoxy-N-methylamino)butyl]phthalimide.

$^1$H-NMR (60MHz, $CDCl_3$);

= 1.66 (m, 4H), 2.56 (s, 3H),
2.62 (t, J=7Hz, 2H),
3.17 (s, 4H), 3.73 (t, J=7Hz, 2H),
7.79 (m, 4H) ppm

(2) Preparation of $N^6$-[4-(N-methoxy-N-methylamino)butyl]adenine by deprotection of the imide, followed by condensation with 6-chloropurine:

1.01 g (3.85 mmol) of the imide obtained in the above Example 3 preparation (1) was dissolved in 15 ml of ethanol and thereto was added 0.231 g (4.06 mmol) of hydrazine hydrate, followed by refluxing for 6 hours and cooling in an ice bath. The resulting solid was filtered off and the filtrate was concentrated to 2 ml by an evaporator on a water bath at 5°C. This solution was mixed with 10 ml of n-butanol and to the mixture were added 0.357 g (2.31 mmol) of 6-chloropurine and 0.67 ml of ethyldiisopropylamine and the mixture was gently refluxed for 6 hours. This was cooled to room temperature and then the solvent was distilled off under reduced pressure. The residue was purified by column chromatography on silica gel (eluent: chloroform/ethanol = 88/12) to give 0.299 g (yield: 31%) of white crystals of $N^6$-[4-(N-methoxy-N-methylamino)butyl]adenine.

Melting point: 181-184°C
Elemental analysis:

| | |
|---|---|
| Molecular formula: | $C_{11}H_{18}N_6O$ |
| Calculated values: | C; 52.78, H, 7.25, N; 33.58 |
| Found : | C; 52.91, H, 7.20, N, 33.42 |

$^1$H-NMR (60 MHz, $CDCl_3$);
= 1.74 (m, 4H), 2.55 (s, 3H),
2.65 (t, J=6Hz, 2H),
3.47 (s, 3H), 3.75 (m, 2H),
6.38 (t, J=6Hz, 1H),
7.88 (m, 1H), 8.32 (s, 1H) ppm

The IR absorption spectrum (KBr method) is shown in Figure 3.
$\nu_{max}$ = $2930^{s}$, $1590^{s}$, $1035^{m}$cm$^{-1}$
UV absorption spectrum
$\lambda_{max}$ = ($H_2O$), 210, 269nm
(0.1N-HC ), 273nm
(0.1N-NaOH), 274nm, $283^{sh}$cm$^{-1}$

## EXAMPLE 4

Preparation of $N^6$-[2-(N-methoxy-N-methylamino)ethyl]adenine hydrochloride:

0.44 g (2 mmol) of $N^6$-[2-(N-methoxy-N-methylamino)ethyl]adenine was dissolved in 30 ml of ethanol and this solution was mixed with 0.2 g (2 mmol) of 36% hydrochloric acid. The solvent was distilled off and the residual crystals were washed thrice with 5 ml of a mixed solvent of methanol and ethanol (5:95) and dried to give 0.48 g (yield: 93%) of the hydrochloride salt of $N^6$-[2-(N-methoxy-N-methylamino)ethyl]adenine.

Melting point: 172-176°C
Elemental Analysis:

| | |
|---|---|
| Molecular formula: | $C_9H_{15}N_6$ ClO |
| Calculated values: | C; 41.78%, H; 5.84%, N; 32.48% |
| | C1; 13.70% |
| Found : | C; 41.79%, H; 5.77%, N; 32.59% |
| | C1; 13.65% |

$^1$H-NMR (90 MHz, DMSO);
= 2.66 (s, 3H), 3.03 (t, J=6Hz, 2H),
3.55 (s, 3H), 3.86 (m, 2H),
8.69 (s, 2H) ppm

The IR absorption spectrum (KBr method) is shown in Figure 4.
$\nu_{max}$ = $2950^{brs}$, $1650^{s}$, $1440^{s}$cm$^{-1}$
UV absorption spectrum
$\lambda_{max}$ = ($H_2O$), 210, 268nm
(0.1N-HCl), 275nm
(0.1N-NaOH), 274nm, $282^{sh}$cm$^{-1}$

EXAMPLE 5

[Assay of cytokinin activity by measurement of chlorophyll content]

Rice seeds (var.: Nankin No. 11) were sown in soil mixed with fertilizer in a seed box and raised for about one month in a greenhouse (25°C in the daytime and 15°C in the night).

A leaf section 1 cm long was cut off from the center of the fourth leaf of the seedlings at the sixth leaf stage. Five leaf sections as one group which had been cut off were floated on the surface of an aqueous solution (2 ml) of the candidate compound or benzyl adenine at a given concentration contained in a glass tubular bottle of 32 mm in inner diameter. This was left to stand in the dark at 30°C for 3 days. The leaf sections were taken out and put in a test tube containing 10 ml of 80% ethanol and the glass tube was immersed in a water bath of 80°C for 20 minutes to extract chlorophyll. After cooling, 80% ethanol was added to make up to 10 ml and absorbance at 665 nm was measured. The results are shown in Table 1. For the untreated group, water was used in place of the candidate solution in all examples.

Senescence retardation was determined according to the degree of chlorophyll retention, that is, calculated using the following formula (in all examples).

$$\text{Senescence retardation (\%)} = \frac{(A) - (B)}{(C) - (B)} \times 100$$

(A) : Absorbance of treated group after 3 days
(B) : Absorbance of untreated group after 3 days
(C) : Absorbance of leaves before treatment.

TABLE 1

The senescence retardation (%) of adenine derivatives.

| Concentration (mg/l) | 0.001 | 0.01 | 0.1 | 1.0 | 10 | 100 |
|---|---|---|---|---|---|---|
| MAED | 14 | 60 | 100 | 95 | - | - |
| MAPD | - | 18 | 50 | 94 | 99 | 91 |
| MABD | - | 1 | 11 | 21 | 77 | 87 |
| Benzyladenine | 17 | 64 | 93 | 93 | - | - |

MAED: $N^6$-[2-(N-methoxy-N-methylamino)ethyl]adenine
MAPD: $N^6$-[3-(N-methoxy-N-methylamino)propyl]adenine
MABD: $N^6$-[4-(N-methoxy-N-methylamino)butyl]adenine
(These are the same in all of the following examples.)

EXAMPLE 6

[Assay for cytokinin activity according to glycine (soybean) hypocotyl section growth test]

Soybean seeds (var.: Enrei) were disinfected with antiformin containing 0.7% effective chlorine for 4 minutes and washed thrice with sterilized water.

Agar (15 ml, 1.6%) was put in a test tube 2.5 cm in diameter. A groove was cut by forceps on the surface of the agar and two soybean seeds treated as above were placed in this groove and kept in the dark at 30°C for 5 days.

In a glass tubular bottle 26 mm in inner diameter was put Miller's medium, to which a given concentration of the candidate compound, benzyladenine or zeatin and 9 ml/l of 2,4-D were added. The central portion of

hypocotyl budding out from the above seeds was cut off to give a section 1 mm thick. Four of the sections as one group were transplanted to the Miller's medium. This was placed in the dark at 30°C for about one month and then the weight of callus formed was measured. For untreated group, the Miller's medium containing 9 mg/l of 2,4-D was used.

TABLE 2

The weight of soybean callus formed (mg/bottle)

| Concentration (mg/l) | 0 | 0.001 | 0.01 | 0.1 | 1.0 | 10 |
|---|---|---|---|---|---|---|
| MAED | - | 97 | 182 | 654 | 823 | 705 |
| MAPD | - | - | 131 | 143 | 841 | 677 |
| MABD | - | - | 51 | 152 | 178 | 824 |
| Benzyladenine | - | 76 | 836 | 674 | - | - |
| t-Zeatin | - | 52 | 382 | 815 | 662 | - |
| Untreated | 22 | - | - | - | - | - |

EXAMPLE 7

[Assay of cytokinin activity to promote synthesis of batacyanin by pig weed (Amaranthus caudatus)]

Two sheets of filter paper were put double in a plastic container (19 x 28 cm) and were wetted with 60 ml of distilled water. Seeds of pig weed were sown on the filter papers and were germinated by keeping them in the dark at 27°C for 3 days. Seedlings of uniform size were chosen and were cut at the upper part of the hypocotyl and the seed coats were removed. This cotyledon with hypocotyl of 3-4 mm in length was used for assay.

Two sheets of filter paper were put double in a glass tubular bottle 32 mm in inner diameter and 1 ml of 0.0065M potassium phosphate buffer solution (pH: 6.3) containing a given concentration of the candidate compound, benzyladenine or zeatin and 0.5 g/l of tyrosine was added therein and ten of the above cotyledon sections were arranged therein and kept at 27°C for 20 hours in the dark. These ten cotyledon sections were put in a test tube containing 3 ml of distilled water and betacyanin was extracted by repeating thrice freezing and thawing. Absorbance of the solution was measured and the amount of the dye was calculated by the difference between the absorbance at 542 nm and at 620 nm. For the untreated group, 0.0065M potassium phosphate buffer solution (pH: 6.3) containing 0.5 g/l of tyrosine was used. The results are shown in Table 3.

TABLE 3

The amount of betacyanin produced (difference between absorbance at 542 nm and 620 nm).

| Concentration (mg/l) | 0 | 0.001 | 0.01 | 0.1 | 1.0 | 10 | 100 |
|---|---|---|---|---|---|---|---|
| MAED | - | 0.040 | 0.058 | 0.121 | 0.214 | - | - |
| MAPD | - | - | - | 0.065 | 0.132 | 0.227 | 0.236 |
| MABD | - | - | - | 0.044 | 0.069 | 0.127 | 0.207 |
| Benzyladenine | - | 0.038 | 0.076 | 0.192 | 0.260 | - | - |
| t-Zeatin | - | 0.040 | 0.062 | 0.198 | 0.242 | - | - |
| Untreated | 0.037 | - | - | - | - | - | - |

EXAMPLE 8

[Assay for absorption and translocation using wheat)

(1) Comparison of activity of MAED and benzyladenine on leaf section of wheat:

Seeds of wheat [var.: Yukichabo (Triticum astivum)] were sown in vermiculite packed in a seed-bed box and were sprouted by keeping them at 28°C for 2 days in the dark. The seedlings wera grown at 25°C for 5 days under irradiation with light of 1500 Lx. When they grew to a length of 12 - 13 cm, a portion having a length of 1 cm was cut off from the leaf in the region 2 - 5 cm from the tip of the first true leaf. Five of the leaf sections as one group were floated with the upper surface of the leaf being uppermost on the surface of aqueous solution (2 ml) of a given concentration of the candidate compound or benzyladenine contained in a glass tubular bottle 32 mm in inner diameter. These were placed at 25°C for 4 days in the dark and thereafter, these leaf sections were put in a test tube containing 10 ml of 80% ethanol and this test tube was immersed in a water bath at 80°C for 20 minutes to extract chlorophyll. After cooling, 80% ethanol was added to make up to 10 ml and absorbance at 665 nm was measured. Senescence retardation was determined according to the degree of retention of chlorophyll. For the untreated group, water was used in place of the aqueous solution of candidate compound. The results are shown in Table 4.

TABLE 4

The senescence retardation (%) of adenine derivatives.

| Concentration (mg/l) | 0.0001 | 0.001 | 0.01 | 0.1 | 1.0 | 10 |
|---|---|---|---|---|---|---|
| MAED | 4 | 14 | 43 | 56 | 58 | 76 |
| Benzyladenine | 7 | 15 | 50 | 55 | 74 | 75 |

(2) Absorption of adenine derivatives from the surface of the leaf of wheat and translocation thereof:

In the same manner as in the above Example 8 test (1), wheat seedlings were grown to a height of

12 - 13 cm and a portion was cut off from the region 4 cm from the tip of the first true leaf and this leaf section was used for this test.

The bottom of a Petri dish 9 cm in diameter was covered with a sheet of circular filter paper which was wetted with 2.5 ml of distilled water. A slide glass was put thereon and ten of the leaf sections were arranged on this slide glass with the upper surface of the leaf being upperward. On the center of the leaf sections was put 10 μl of the solution prepared by dissolving the candidate compound or benzyladenine in a 0.2% aqueous solution of Tween 80 at a given concentration. The Petri dish was covered and left at 25°C for 5 days in the dark. In the part where the candidate solution was absorbed and translocated around the position on which the solution was put, the length of the portion where chlorophyll was retained, namely, the length of the remaining green zone was measured. The results are shown in Table 5. At the same concentration, MAED expanded the green zone in more widely than benzyladenine and this means that MAED is superior in absorbability and translocation.

TABLE 5

The length of the green zone (mm) (average in 10 leaves).

| Concentration (mg/l) | 0.1 | 1.0 | 10 |
|---|---|---|---|
| MAED | 9.9 | 19.8 | 28.8 |
| Benzyladenine | 9.6 | 14.2 | 16.9 |

(3) Absorption of the adenine derivatives from the base of wheat and translocation thereof:

Wheat seedlings were grown to a height of 14-15 cm in the same manner as in the above Example 8 test (1) and the shoot was cut off at the base. These sections were used for the test.

A solution (10 ml) containing 10 mg/l of the candidate compound or benzyladenine was poured into a test tube 2.5 cm in diameter and five of the wheat shoots as one group were inserted in one test tube containing the candidate solution and left at 25°C for 18 hours under irradiation with light of 1500 Lx. Thereafter, each of the shoots was cut at 5-6 cm, 8-9 cm and 11-12 cm from the above cut end and five of the respective sections as one group were floated on the surface of distilled water (2 ml) in a glass tubular bottle 32 mm in inner diameter with the upper surface of the leaves being upperward. These were left at 25°C for 3 days in the dark and thereafter, the leaf sections were put in a test tube containing 10 ml of 80% ethanol. This test tube was immersed in a water bath at 80°C for 20 minutes to extract chlorophyll. After cooling, 80% ethanol was added to make up to 10 ml and absorbance of the solution at 665 nm was measured. In the same manner as in the above Example 8 test (1), senescence retardation was obtained. The results are shown in Table 6.

TABLE 6

The senescence retardation (%) of adenine derivatives.

| Region of leaf section | 5-6 (cm) | 8-9 (cm) | 11-12 cm |
|---|---|---|---|
| MAED | 37 | 34 | 20 |
| Benzyladenine | 17 | 21 | 14 |

It can be seen from Table 6 that MAED showed a more pronounced effect of senescence retardation than benzyladenine in all regions of the first true leaf of wheat and thus MAED has superior absorbability and translocation.

EXAMPLE 9

(Test on effect to promote photosynthesis)

Soybeans (var.: Enrei) were shown in a pot and raised in a phytotron under natural light set at 25°C in the daytime and 20°C in the night and 70-80% in humidity. The seedlings at the fourth leaf stage (34th day after sowing) were subjected to application with an aqueous solution containing 1 ppm of MAED and 200 ppm of Tween 20 as a surface active agent in such a manner that the surfaces of the leaves were wetted. On 30th day after application, the third leaf was subjected to measurement of photosynthetic rate by $CO_2$ measuring method using an assimilation box. The absorption rate of $CO_2$ is shown in Figure 5.

EXAMPLE 10

Water solubility of adenine derivatives of the present invention and representative known cytokinin substances was measured and the results are shown in Table 7. As shown in Table 7, the adenine derivatives of the present invention show far higher solubility than the known cytokinin substances.

TABLE 7

| Substances | Solubility (g/100 g $H_2O$) (20°C) |
|---|---|
| MAED | 9.9 |
| MAPD | 13.6 |
| MABD | 5.3 |
| Benzyladenine | $3 \times 10^{-3}$ |
| t-Zeatin | 0.14 |
| Kinetin | $2 \times 10^{-3}$ |

EXAMPLE 11

A liquid formulation was prepared by dissolving 0.1 g of MAED and 10.0 g of SORPOL-8222 (nonionic surface-active agent manufactured by Toho Chemical Co. Ltd.) in 89.9 g of water.

EXAMPLE 12

An emulsifiable concentrate was prepared by dissolving 2 g of MAED and 38 g of AEROL CT-1 (anionic surface-active agent manufactured by Toho Chemical Co. Ltd.) in 60 g of dimethyl sulfoxide.

EXAMPLE 13

A wettable powder was prepared by pulverizing 10 g of MAED, 15 g of white carbon, 3 g of calcium lignin sulfonate, 1 g of polyoxyethylenenonylphenyl ether, 5 g of diatomaceous earth, and 66 g of clay and homogeneously mixing them.

EXAMPLE 14

Young seedlings of rice (var.: Koshihikari) were planted in a paddy field on 20th April. The MAED liquid formulation prepared in Example 11 was diluted with water to the given concentrations as shown in Table 8 and 100 l/10a of each solution was applied to leaves and stems on 4th August at the boot stage and on 19th August at the initial ripening stage (the tenth day after heading), respectively.

As a comparative example, an emulsifiable concentrate (hereinafter referred to as "BA emulsifiable concentrate") was prepared by dissolving 2 g of benzyladenine and 38 g of AEROL CT-1 (an anionic surface-active

agent manufactured by Toho Chemical Co. Ltd.) in 60 g of dimethylsulfoxide. It was applied at the same stage in the same concentration with the same total amount as in application of the MAED liquid formulation.

One experimental plot had an area of 6 $m^2$ and three plots were used for each treatment. The rice plants were harvested on 19th September which corresponded to the 41st day after heading and the husked rice weight and thousand-kernel weight of husked rice were measured.

The results are shown in Table 8.

TABLE 8

| Chemicals | Treating stage | Concent-ration (ppm) | Husked rice weight (ratio to untreated rice) (%) | Thousand-kernel weight of husked rice (ratio to un-treated rice) (%) |
|---|---|---|---|---|
| MAED liquid formulation | Boot stage | 0.3 | 105 | 101 |
| | | 1 | 108 | 102 |
| | | 3 | 109 | 102 |
| | Tenth day after heading | 0.3 | 108 | 102 |
| | | 1 | 110 | 103 |
| | | 3 | 109 | 102 |
| BA emulsi-fiable con-centrate | Boot stage | 0.3 | 100 | 100 |
| | | 1 | 102 | 100 |
| | | 3 | 103 | 100 |
| | Tenth day after heading | 0.3 | 100 | 100 |
| | | 1 | 103 | 100 |
| | | 3 | 104 | 101 |
| Untreated | - | | 100 | 100 |

The husked rice weight of the untreated section was 536 kg/10a and the thousand-kernel weight of the husked rice was 21.0 g.

EXAMPLE 15

Seeds of winter wheat (var.: Norin No. 61) were sown in a field on 20th October and leaves and stems were subjected to application with 100 l/10a of the diluted MAED liquid formulation and the diluted BA emulsifiable concentrate as a comparative example, prepared in the same manner as in Example 14, on 20th April corresponding to the most growing stage and on 10th May corresponding to the initial ripening stage, respectively.

On the 235th day after sowing, the crops were harvested and the grain weight was measured.

The results are shown in Table 9.

TABLE 9

| Chemicals | Treating stage | Concentration (ppm) | Grain weight (ratio to untreated ones) (%) |
|---|---|---|---|
| MAED liquid formulation | The most growing stage | 0.1 | 111 |
| | | 0.3 | 110 |
| | | 1 | 116 |
| | The initial ripening stage | 0.1 | 117 |
| | | 0.3 | 119 |
| | | 1 | 110 |
| BA emulsifiable concentrate | The most growing stage | 0.1 | 100 |
| | | 0.3 | 100 |
| | | 1 | 103 |
| | The initial ripening stage | 0.1 | 100 |
| | | 0.3 | 104 |
| | | 1 | 105 |
| Untreated | - | - | 100 |

The grain weight per 10a of the untreated group was 470 kg.

EXAMPLE 16

Seeds of soybean (var.: Enrei) were sown in a field on 15th May and leaves and stems were subjected to application with 100 l/10a of the diluted MAED liquid formulation and the diluted emulsifiable concentrates as a comparative example, prepared in the same manner as in Example 14, on 3rd July corresponding to the beginning of flowering and on 7th August corresponding to the initial ripening stage, respectively.

One experimental plot had an area of 12 $m^2$ and four replicate plots were used for each treatment. On the 132nd day after sowing, the crops were harvested and the grain weight was measured.

The results are shown in Table 10.

TABLE 10

| Chemicals | Treating stage | Concentration (ppm) | Grain weight (ratio to untreated ones) (%) |
|---|---|---|---|
| MAED liquid formulation | The beginning of flowering | 0.3<br>1 | 110<br>116 |
| | The initial ripening stage | 0.3<br>1 | 107<br>112 |
| BA emulsifiable concentrate | The beginning of flowering | 0.3<br>1 | 102<br>104 |
| | The initial ripening stage | 0.3<br>1 | 103<br>103 |
| Untreated | - | - | 100 |

The grain weight per 10a of the untreated group was 372 kg.

## Claims

1. Adenine derivatives represented by the formula I and salts thereof represented by the formula II:

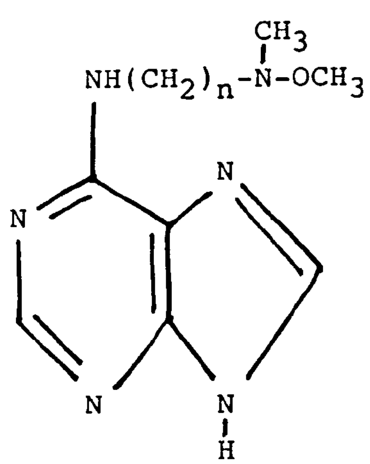

(I)

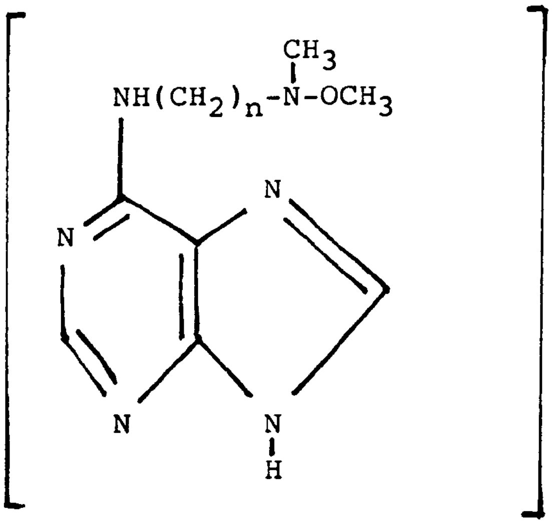

• X (II)

wherein n is an integer of from 2 to 4 and X is an equivalent of hydrochloric acid, sulfuric acid, nitric acid, phosphoric acid, formic acid or acetic acid.

2. An adenine derivative according to Claim 1 which is represented by the formula (1):

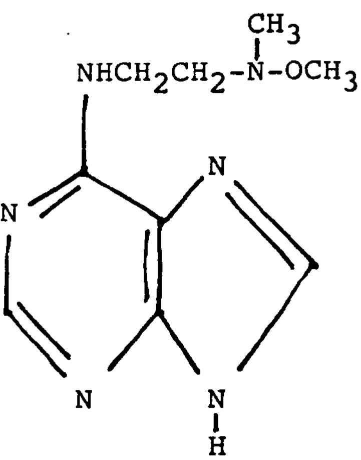

(1)

or a salt of adenine derivative represented by the formula (5):

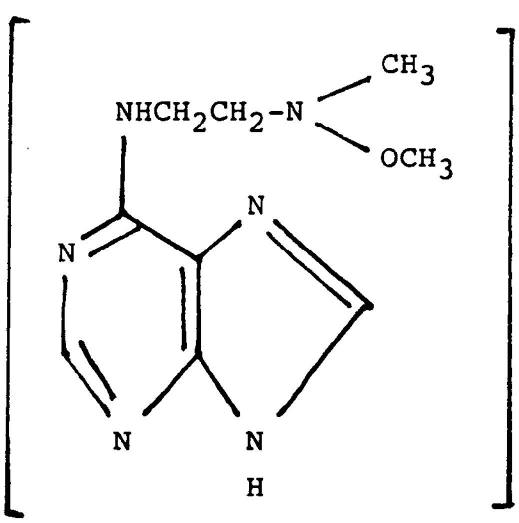

• X (5)

wherein X is an equivalent of hydrochloric acid, sulfuric acid, nitric acid, phosphoric acid, formic acid or acetic acid.

3. An adenine derivative according to Claim 1 which is represented by the formula (2):

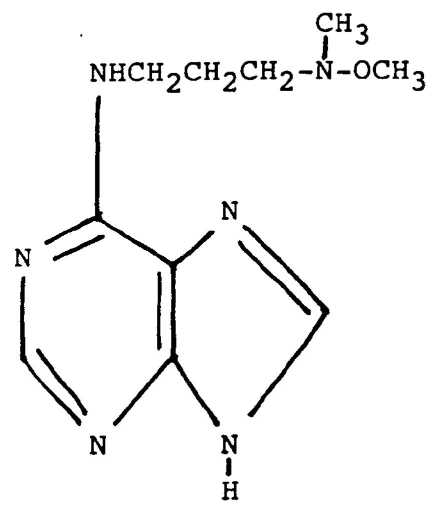

(2)

4. An adenine derivative according to Claim 1 which is represented by the formula (3):

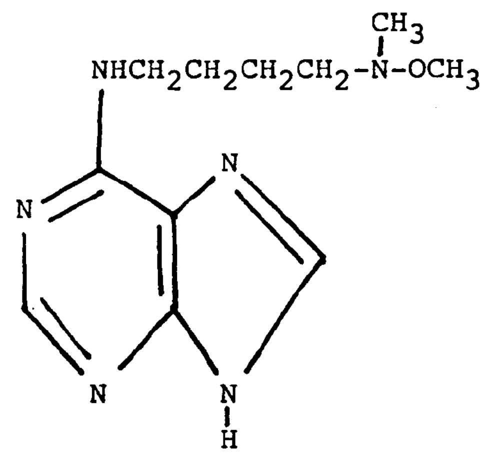

(3)

5. A salt of adenine derivative according to Claim 1 which is represented by the formula (4):

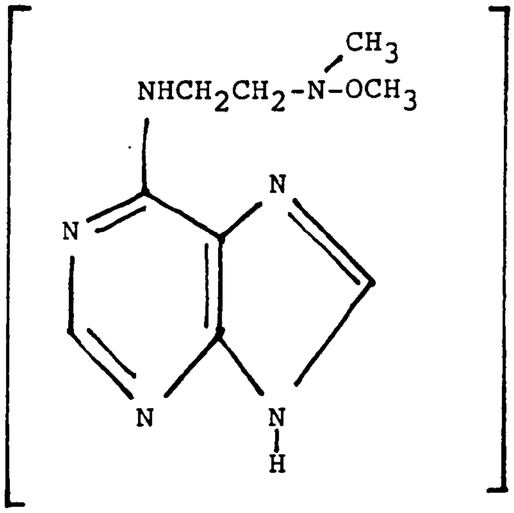

· HCl (4)

6. An agent for increasing the yield of crops which contains, as an active ingredient, a compound or an adenine derivative as claimed in any one of Claims 1 to 5.

7. An agent as claimed in Claim 6 which increases the yield of leguminous crops or gramineous crops.

8. An agent as claimed in Claim 7 in the form of a liquid formulation.

9. A method for increasing yield of crops which comprises applying an agent as claimed in Claim 6, 7 or 8 to crops to be treated.

10. A method as claimed in Claim 9 which comprises applying the agent to leaves and stems of leguminous crops or gramineous crops.

11. A method as claimed in Claim 9 wherein the dosage of the agent is 0.03-3 g/10a in terms of the adenine derivative of the formula (1).

12. A method as claimed in Claim 9 which comprises applying the agent to leguminous crops during the period of from the flower bud appearing stage to the grain ripening stage.

13. A method as claimed in Claim 9 which comprises applying the agent to gramineous crops during the period of the panicle formation stage to the ripening stage.

14. The use of a compound as claimed in any one of Claims 1 to 5 in the preparation of an agent having cytokinin activity for treating plants.

**Patentansprüche**

1. Adeninderivate, dargestellt durch Formel I, und deren Salze, dargestellt durch Formel II:

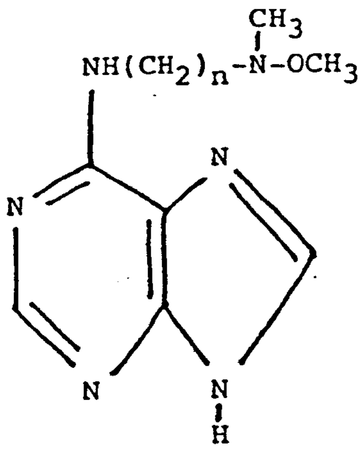

(I)

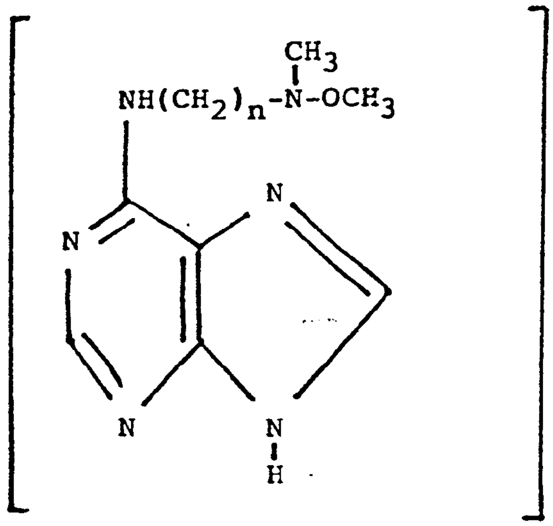

· X (II)

wobei n eine ganze Zahl von 2 bis 4 ist und X ein Äquivalent von Salzsäure, Schwefelsäure, Salpetersäure, Phosphorsäure, Ameisensäure oder Essigsäure darstellt.

2. Ein Adeninderivat nach Anspruch 1, dargestellt durch Formel (1):

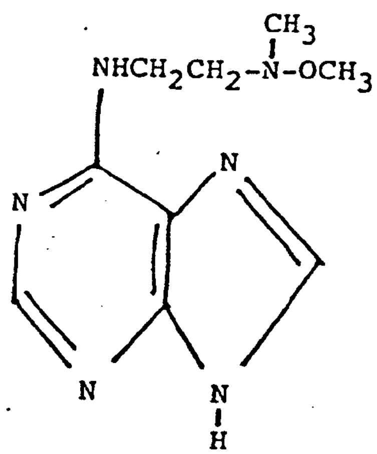

(1)

oder ein Salz des Adeninderivats, dargestellt durch Formel (5):

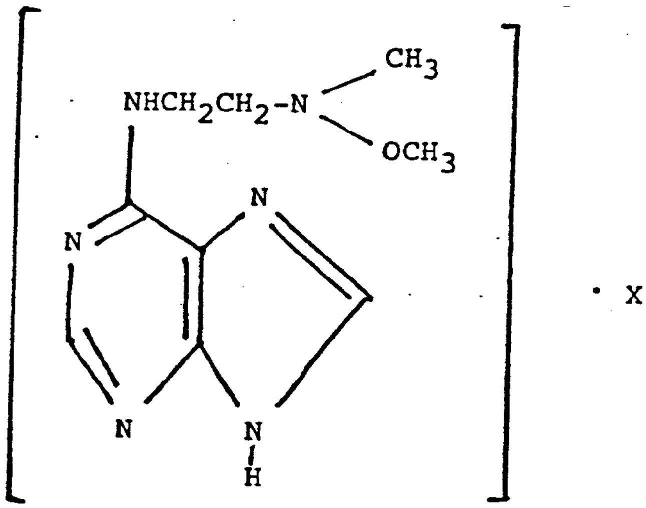

(5)

wobei X ein Äquivalent von Salzsäure, Schwefelsäure, Salpetersäure, Phosphorsäure, Ameisensäure oder Essigsäure ist.

3. Ein Adeninderivat nach Anspruch 1, dargestellt durch Formel (2):

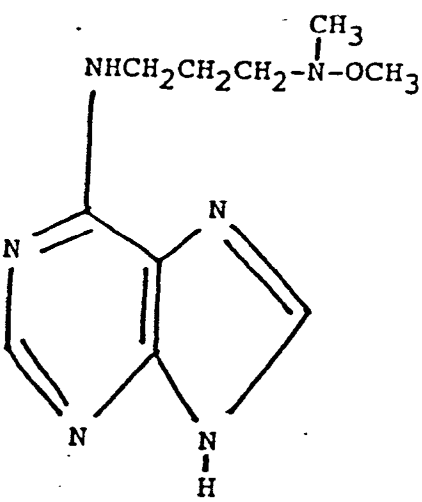

(2)

4. Ein Adeninderivat nach Anspruch 1, dargestellt durch Formel (3):

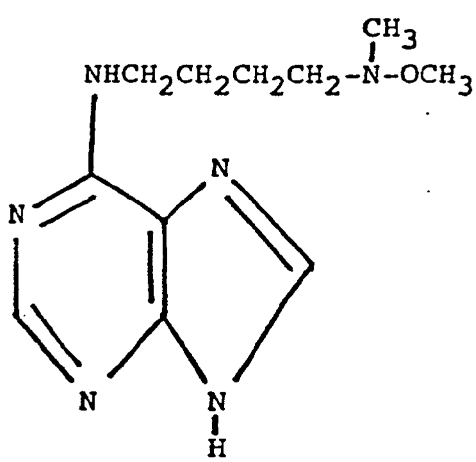

(3)

5. Ein Salz eines Adeninderivates nach Anspruch 1, dargestellt durch Formel (4):

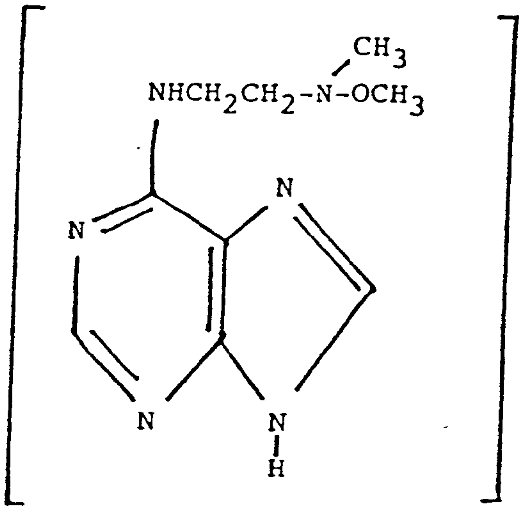

• HCl (4)

6. Ein Mittel zur Erhöhung der Ausbeute an Feldfrüchten, welches als einen aktiven Bestandteil eine Verbindung oder ein Adeninderivat, wie in irgendeinem der Ansprüche 1 bis 5 beansprucht, enthält.

7. Ein Mittel wie in Anspruch 6 beansprucht, welches die Ausbeute an hülsentragenden Feldfrüchten oder grasartigen Feldfrüchten erhöht.

8. Ein Mittel wie in Anspruch 7 beansprucht in Form einer flüssigen Formulierung.

9. Eine Methode zur Erhöhung der Ausbeute an Feldfrüchten, umfassend das Anwenden eines Mittels, wie in Anspruch 6, 7 oder 8 beansprucht, auf zu behandelnde Feldfrüchte.

10. Eine Methode wie in Anspruch 9 beansprucht, umfassend das Anwenden des Mittels auf Blätter und Stiele von hülsentragenden Feldfrüchten oder grasartigen Feldfrüchten.

11. Eine Methode wie in Anspruch 9 beansprucht, wobei die Dosierung des Mittels 0,03 - 3 g/10Ar, bezogen auf das Adeninderivat der Formel (1), beträgt.

12. Eine Methode wie in Anspruch 9 beansprucht, umfassend das Anwenden des Mittels auf hülsentragende Feldfrüchte während der Zeit vom Beginn des Blütenknospenstadiums bis zum Kornreifestadium.

13. Eine Methode wie in Anspruch 9 beansprucht, umfassend das Anwenden des Mittels auf grasartige Feldfrüchte während der Zeit des Rispenbildungsstadiums bis zum Reifestadium.

14. Die Verwendung einer Verbindung wie in irgendeinem der Ansprüche 1 bis 5 beansprucht in der Herstellung eines Mittels mit Cytokininaktivität zur Behandlung von Pflanzen.

## Revendications

1. Dérivés de l'adénine représentés par la formule I et leurs sels représentés par la formule II :

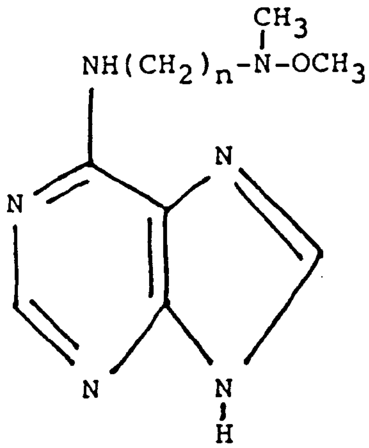

(I)

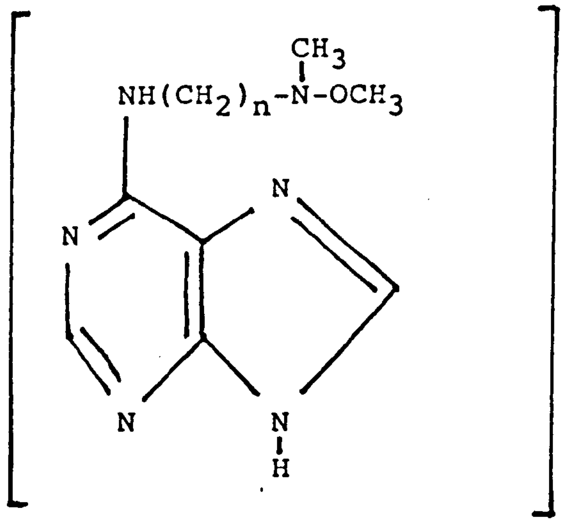

X (II)

dans laquelle n est un nombre entier de 2 à 4 et X est un équivalent d'acide chlorhydrique, d'acide sulfurique, d'acide nitrique, d'acide phosphorique, d'acide formique ou d'acide acétique.

**2.** Dérivé de l'adénine suivant la revendication 1, qui est représenté par la formule (1) :

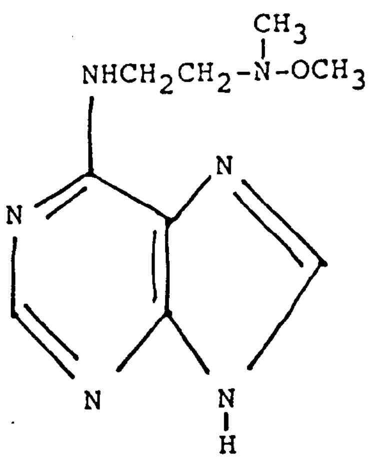

(1)

ou un sel de dérivé d'adénine représenté par la formule (5) :

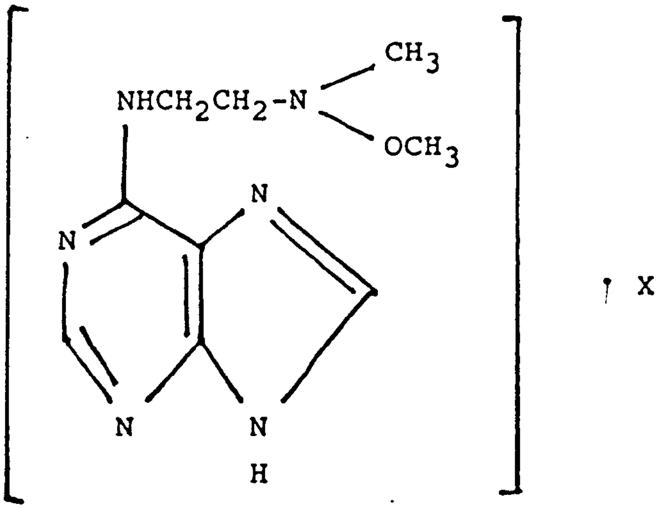

(5)

dans laquelle X est un équivalent d'acide chlorhydrique, d'acide sulfurique, d'acide nitrique, d'acide formique ou d'acide acétique.

**3.** Dérivé de l'adénine suivant la revendication 1, qui est représenté par la formule (2).

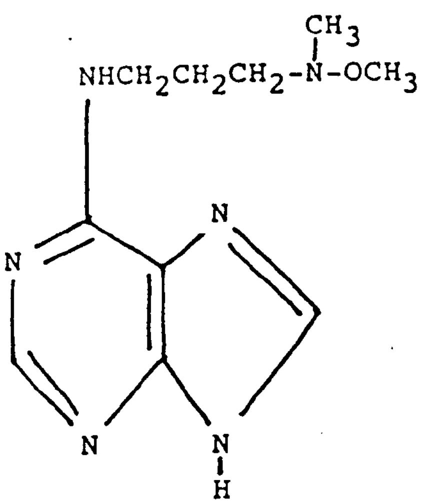

(2)

**4.** Dérivé de l'adénine suivant la revendication 1, 15 qui est représenté par la formule (3) :

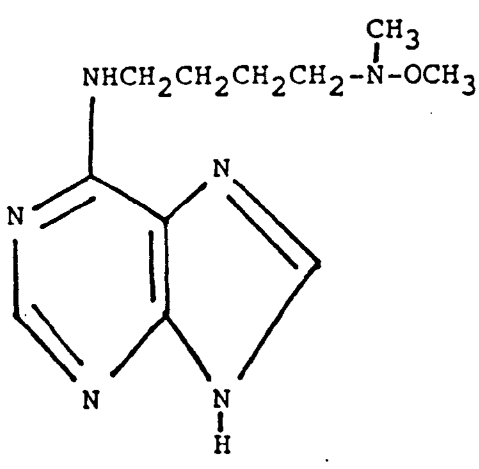

(3)

**5.** Sel d'un dérivé de l'adénine suivant la revendication 1, qui est représenté par la formule (4) :

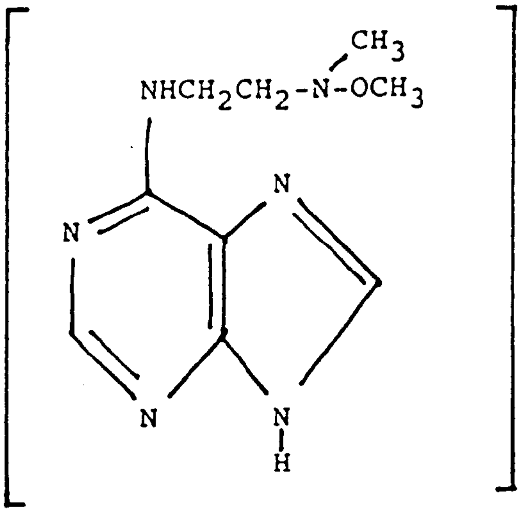

· HCl (4)

**6.** Agent pour augmenter le rendement de cultures, qui contient, comme ingrédient actif, un composé ou un dérivé de l'adénine tel que revendiqué selon l'une quelconque des revendications 1 à 5.

**7.** Agent suivant la revendication 6, qui augmente le rendement de culture de légumes ou de cultures de graminées.

**8.** Agent suivant la revendication 7 sous la forme d'une formulation liquide.

**9.** Procédé pour augmenter le rendement de culture, qui consiste à appliquer un agent tel que revendiqué à la revendication 6, 7 ou 8 à des cultures à traiter.

**10.** Procédé suivant la revendication 9, qui consiste à appliquer l'agent aux feuilles et aux tiges de cultures de légumes ou de cultures de graminées.

**11.** Procédé suivant la revendication 9, dans lequel le dosage de l'agent est de 0,03 à 3 g/10a exprimé en dérivé de l'adénine de formule (1).

**12.** Procédé suivant la revendication 9, qui consiste à appliquer l'agent à des cultures de légumes pendant la période allant du stade d'apparition du bouton floral au stade de maturité du grain.

**13.** Procédé suivant la revendication 9, qui consiste à appliquer l'agent à des cultures de graminées pendant la période allant du stade de formation du panicule au stade de maturité.

**14.** Utilisation d'un composé suivant l'une quelconque des revendications 1 à 5 pour la préparation d'un agent ayant une activité cytokinine en vue de traiter des plantes.

FIG. 1

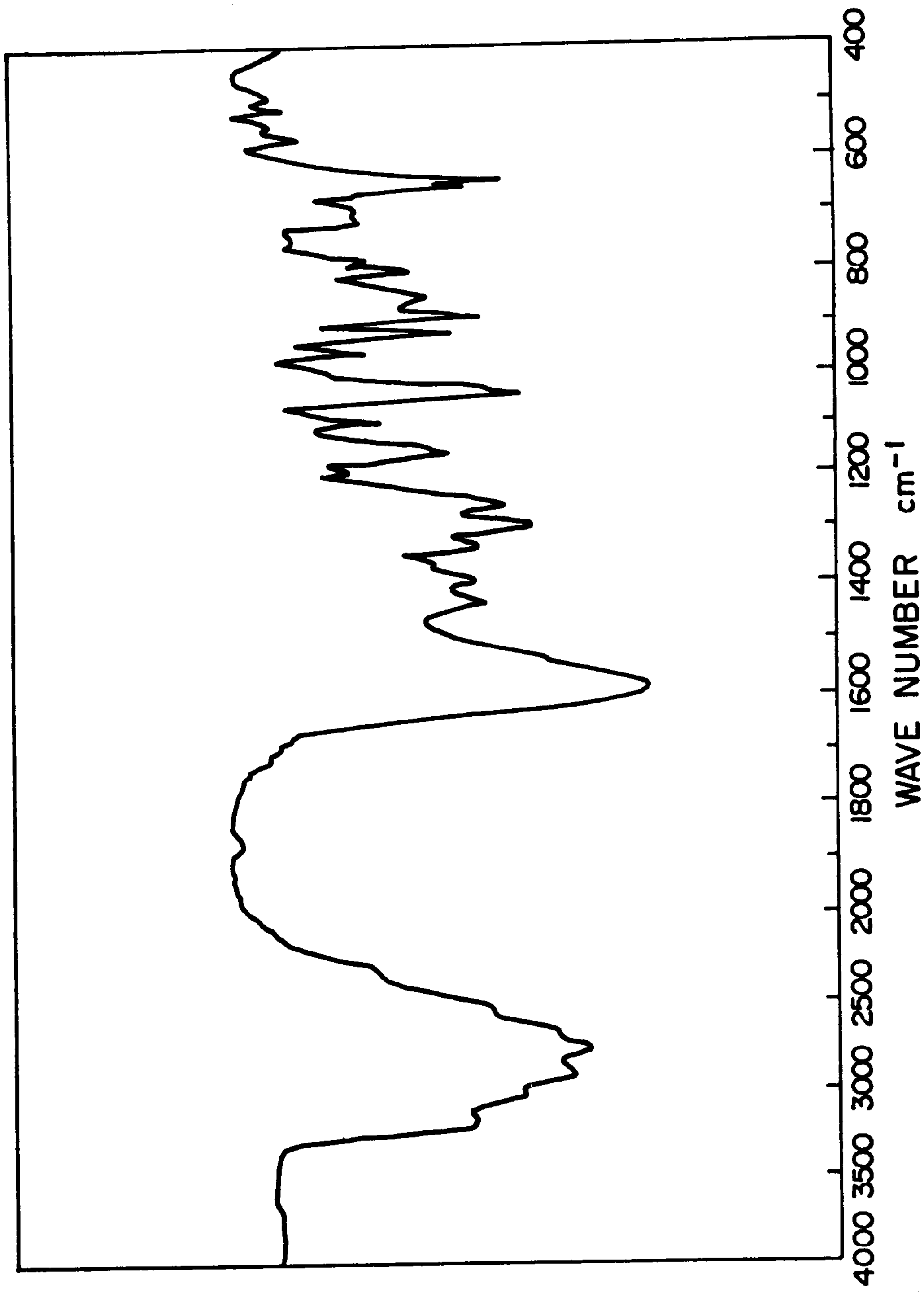

400
600
800
1000
1200
1400
1600
1800
2000
2500
3000
3500
4000
WAVE NUMBER cm$^{-1}$

FIG. 2

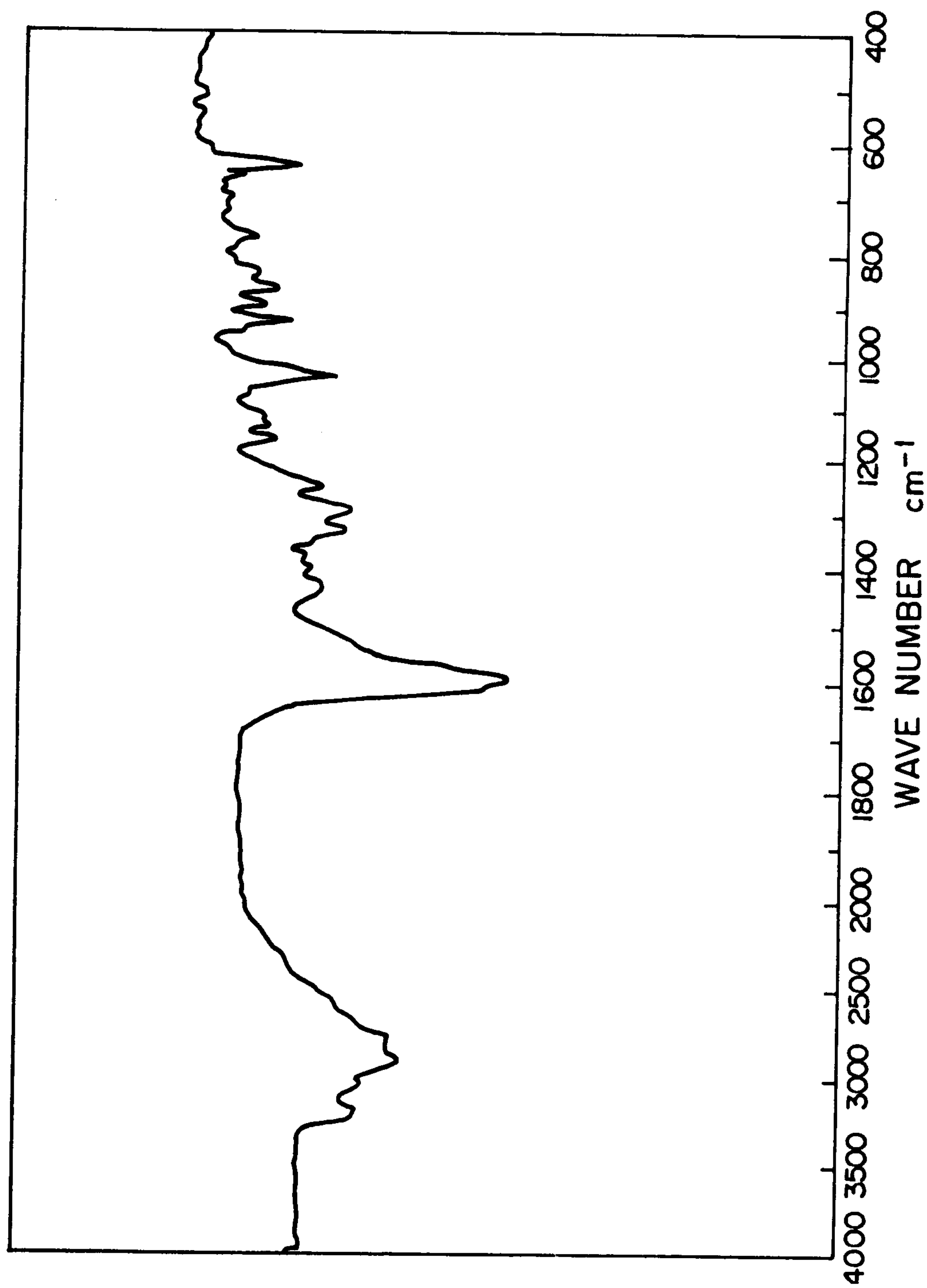
4000
3500
3000
2500
2000
1800
1600
1400
1200
1000
800
600
400
WAVE NUMBER cm⁻¹

FIG. 3

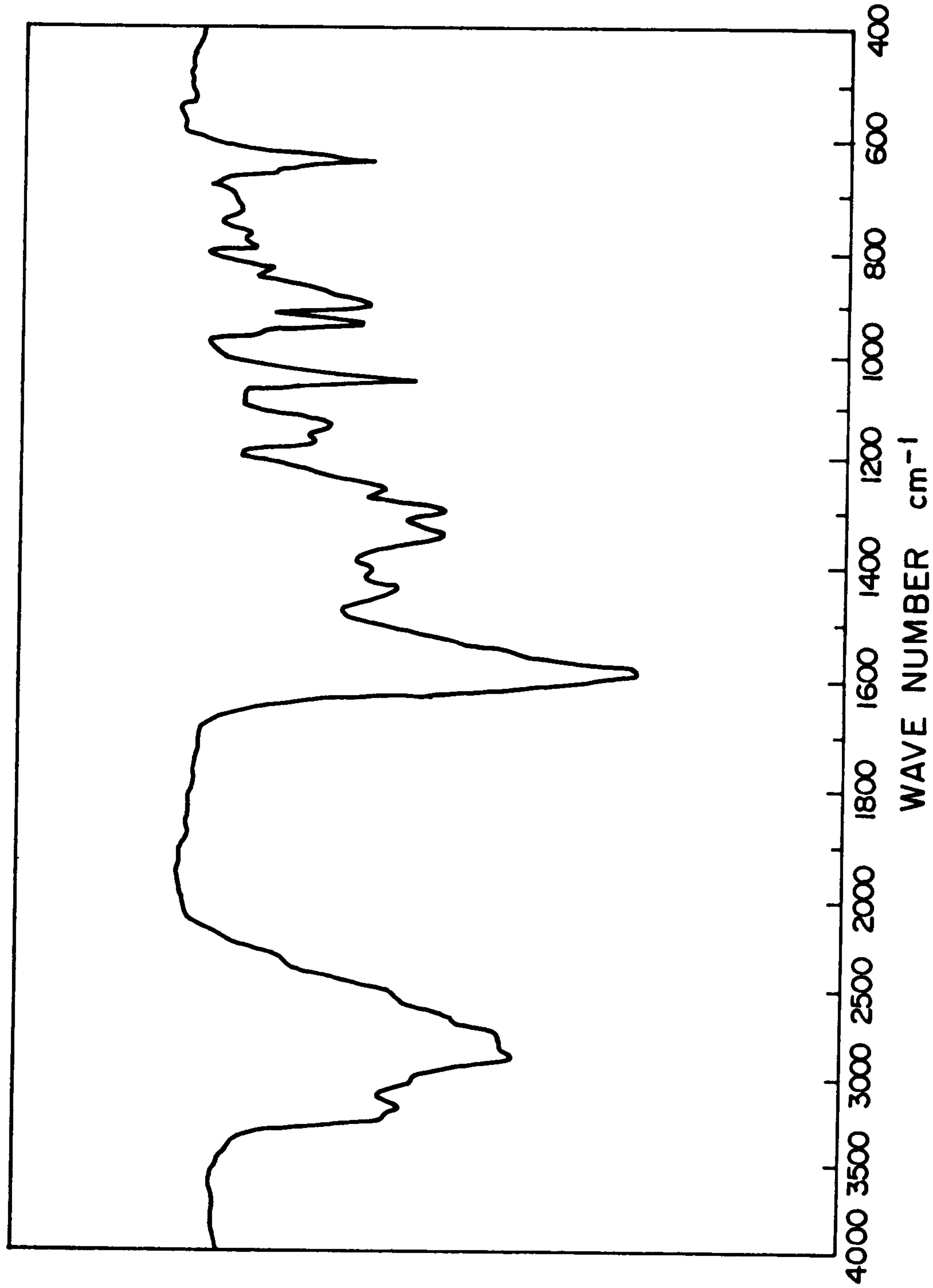
400
600
800
1000
1200
1400
1600
1800
2000
2500
3000
3500
4000
WAVE NUMBER cm$^{-1}$

FIG. 4

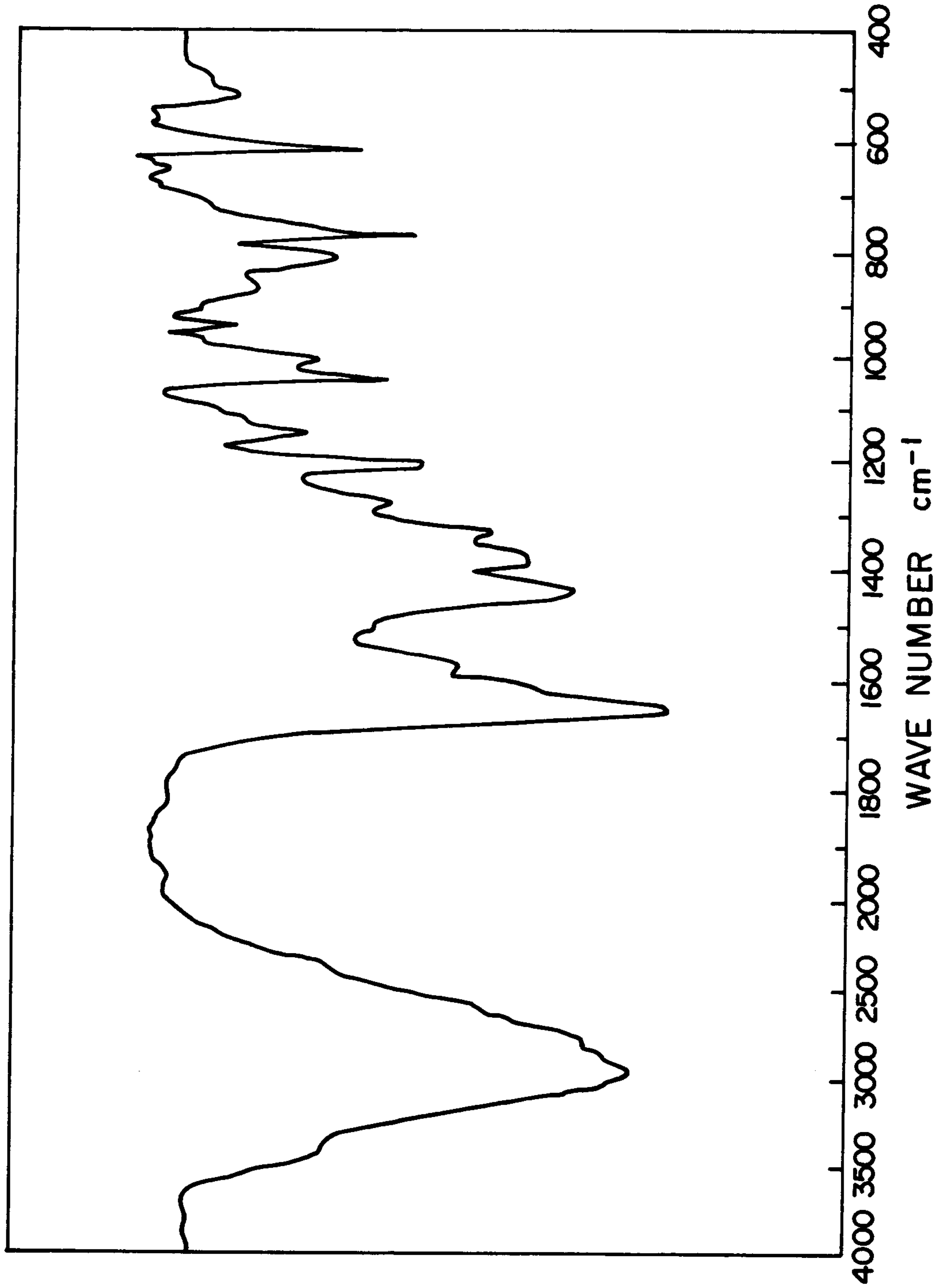
4000
3500
3000
2500
2000
1800
1600
1400
1200
1000
800
600
400
WAVE NUMBER cm-1

FIG. 5

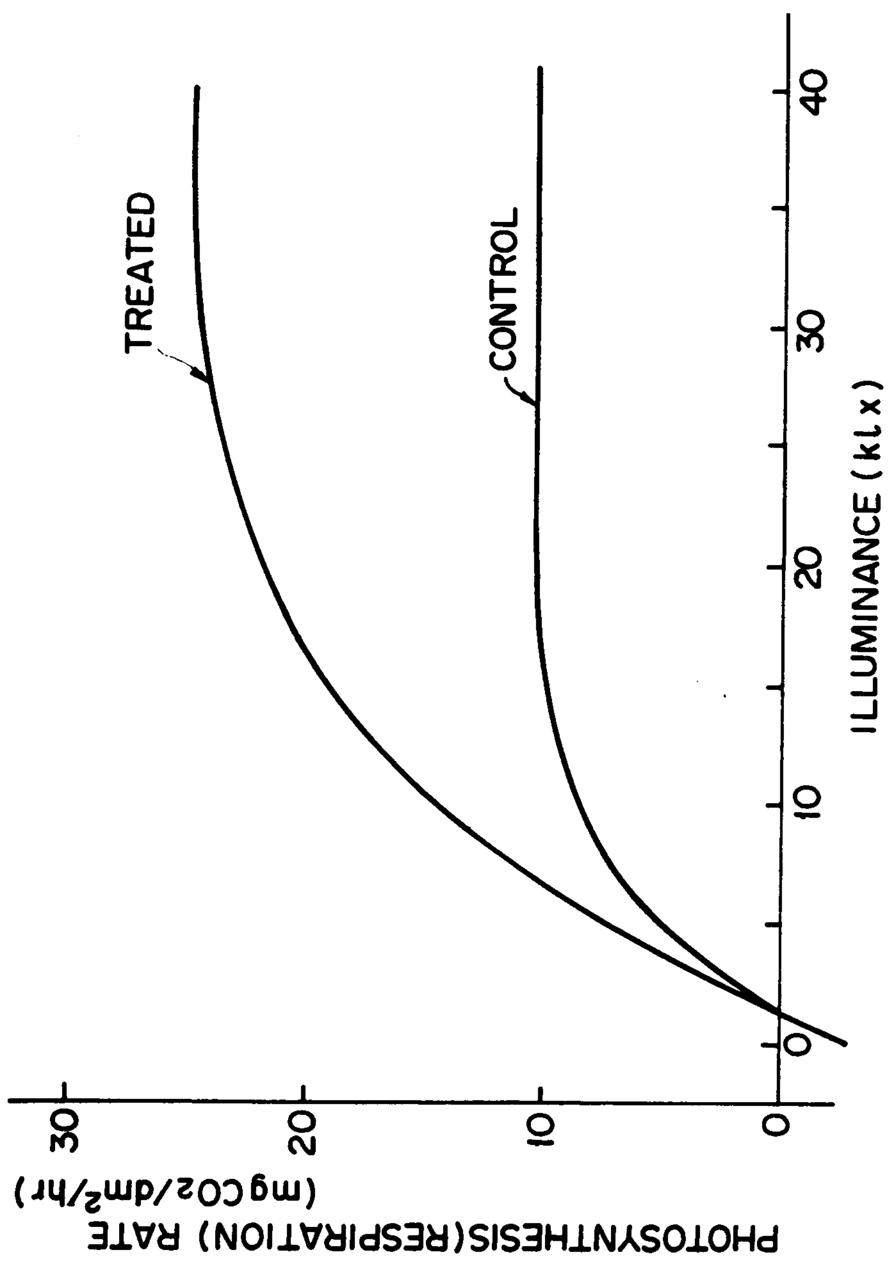
TREATED
CONTROL
ILLUMINANCE (klx)
0
10
20
30
40
PHOTOSYNTHESIS(RESPIRATION) RATE
(mg $CO_2$/$dm^2$/hr)
0
10
20
30